# FASCICULE DE BREVET EUROPEEN

(11) **EP 2 395 067 B1**
(45) Date de publication et mention de la délivrance du brevet: **05.09.2018**
(21) Numéro de dépôt: 11290183.0
(22) Date de dépôt: 12.04.2011
(51) Int. Cl.: C10G 59/02, C10G 35/12

(54) **Procédé de reformage catalytique avec recyclage de l'effluent de réduction du catalyseur en amont du premier réacteur et recyclage du gaz de recyclage sur l'avant dernier réacteur de la série**
Katalytisches Reformierverfahren mit Rückführung der Abgase aus der Katalysatorreduzierung vor dem ersten Reaktor und Rückführung der Abgase aus der Reformierung in den vorletzten Reaktor der Reihe
catalytic reforming process comprising the recycling of the catalyst reduction effluent upstream of the first reactor and the recycling of gaseous reforming effluent to the penultimate reactor of the series

(30) Priorité: 09.06.2010 FR 1002425
(43) Date de publication de la demande: 14.12.2011
(73) Titulaire: IFP Energies nouvelles, 92500 Rueil-Malmaison (FR)
(72) Inventeur: Sanchez, Eric, 69230 Saint Genis Laval (FR)

(56) Documents cités:
- FR-A1- 2 801 605
- US-A- 3 761 390
- US-A- 3 992 465
- US-A- 5 196 110

## Description

### DOMAINE DE L'INVENTION

L'invention concerne le domaine du procédé de reformage catalytique des essences, et plus généralement les procédés en lit mobile. Elle s'applique plus spécifiquement au reformage régénératif ou à la production de BTX (Benzène, Toluène , Xylènes) avec régénération continue du catalyseur.

Le procédé de reformage catalytique des essences fait appel à une zone réactionnelle comportant une série de 3 ou 4 réacteurs en série, travaillant en lit mobile, et présente une zone de régénération du catalyseur qui comprend elle même un certain nombre d'étapes, dont une étape de combustion du coke déposé sur le catalyseur dans la zone réactionnelle, une étape d'oxychloration, et une étape finale de réduction du catalyseur à l'hydrogène. Après la zone de régénération, le catalyseur est réintroduit en tête du premier réacteur de la zone réactionnelle.

L'invention concerne plus précisément un nouveau procédé de reformage catalytique des essences comportant un recyclage de l'effluent de l'étape de réduction du catalyseur en amont du premier réacteur de la zone réactionnelle et une injection de l'hydrogène de recyclage au dernier ou avant dernier réacteur afin de maintenir une pression partielle d'hydrogène plus importante sur ces derniers réacteurs et ainsi de limiter la désactivation du catalyseur en fin de zone réactionnelle.

Cette nouvelle disposition présente plusieurs avantages :
- Elle diminue, voire élimine la réintroduction d'eau dans le système réactionnelle,
- Elle favorise les réactions de déshydrogénation des naphtènes et de deshydrocyclisation des paraffines contenus dans la charge, c'est à dire celles réalisées dans les deux premiers réacteurs grâce à un abaissement de la pression partielle en hydrogène dans ces deux réacteurs, abaissement qui est thermodynamiquement favorable aux réactions en question,
- Elle modifie également favorablement la répartition de l'hydrogène entre les différents réacteurs en augmentant le rapport H2/HC sur le ou les deux derniers réacteurs qui sont précisément ceux dans lesquels le coke a préférentiellement tendance à se former.

### EXAMEN DE L'ART ANTERIEUR

Selon l'art antérieur, l'effluent de réduction d'une unité de reformage catalytique est généralement envoyé, soit à l'aspiration du compresseur de recontactage de la section de purification de l'hydrogène, soit au réseau "fuel gaz" de la raffinerie, c'est à dire au réseau de gaz utilisé comme combustible dans diverses unités ou fours de la raffinerie.

L'effluent de réduction peut aussi être envoyé en totalité ou en partie à l'entrée du ballon séparateur pour ajuster la quantité d'eau dans le gaz de recycle.

Les schémas de la zone de purification de l'art antérieur ne sont pas modifiés par la présente invention qui concerne essentiellement la zone réactionnelle.

Le brevet FR 2 801 604 enseigne un procédé de production d'aromatiques à l'aide d'un catalyseur circulant en lit mobile, procédé qui comprend au moins deux étapes caractérisées par un certain rapport (H2)/(HC), H2 représentant la quantité d'hydrogène introduit dans ladite étape, et HC représentant la quantité de charge entrant dans la dite étape.

Dans le brevet précité l'étape de réduction du catalyseur est également caractérisée par certaines valeurs du rapport H2/HC. Les valeurs des rapports H2/HC des deux étapes réactionnelles et celle de l'étape de réduction du catalyseur sont liées par une relation d'inégalité. Cependant dans tous les cas, l'hydrogène de recyclage est envoyé au premier réacteur de la section réactionnelle et donc ne conduit pas à favoriser les réactions recherchées dans ce procédé qui sont essentiellement les réactions de déshydrogènation des naphtènes ou de déshydrocyclisation des paraffines grandement favorisées par les faibles pressions partielle d'hydrogène.

Le brevet FR 2 801 605 enseigne un procédé de production d'aromatiques à l'aide d'un catalyseur circulant en lit mobile qui comprend une étape de réduction dudit catalyseur en présence d'un gaz de recyclage introduit en quantité telle que la quantité d'hydrogène pur amenée à l'étape de réduction du catalyseur est comprise entre 1 et 10 kg/kg de catalyseur.

La demande FR 09/02.802 décrit un circuit de recyclage de l'effluent de la zone de réduction sur le ou les derniers réacteurs de la zone réactionnelle. Ce recyclage ne modifie pas le niveau de pression partielle en hydrogène au premier réacteur qui est précisément l'action recherchée dans la présente demande.

Aucun de ces trois documents, que l'on peut considérer comme représentant l'art antérieur le plus proche, ne divulgue de manière précise la réintroduction de l'effluent issu de l'étape de réduction du catalyseur en tête du premier réacteur et/ou l'injection de l'effluent issu du ballon séparateur du compresseur de recycle (RCY), communément appelé gaz de recycle, en tête du dernier ou de l'avant dernier réacteur de la zone réactionnelle d'une unité de reformage catalytique des essences. La combinaison de ces deux flux conduit à une amélioration des rendements de l'unité, notamment sur le reformat défini comme les hydrocarbures ayant plus de 4 atomes de carbone et noté C4+.

### DESCRIPTION SOMMAIRE DES FIGURES

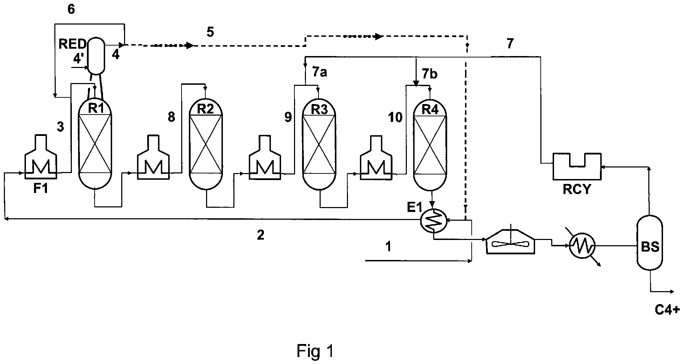

La figure 1 représente une vue générale d'une unité de reformage catalytique comprenant 4 réacteurs en série et une zone de régénération du catalyseur.

Le circuit du catalyseur est marqué en traits plus épais.

La figure 1 permet de visualiser le recyclage de l'effluent de réduction (4) pour partie en tête du premier réacteur, et pour partie à l'entrée de l'échangeur charge effluent, ainsi que la réintroduction du gaz de recyclage (7) issu du compresseur de recyclage (RCY) en tête du dernier (R4) et de l'avant dernier réacteur (R3).

### DESCRIPTION SOMMAIRE DE L'INVENTION

La présente invention concerne un procédé de reformage catalytique d'une charge d'essence d'intervalle de distillation compris entre 60°C et 250°C dans une unité de reformage catalytique en lit mobile comprenant une zone réactionnelle à quatre réacteurs en série selon la revendication 1.

La présente invention peut se définir comme un procédé de reformage catalytique d'une essence d'intervalle de distillation compris entre 60°C et 250°C faisant appel à une unité de reformage catalytique en lit mobile comprenant au moins quatre réacteurs en série, et une zone de régénération dudit catalyseur, dans lequel l'effluent de l'étape de réduction du catalyseur faisant partie de la zone de régénération du catalyseur, est recyclé pour partie à l'entrée de l'échangeur charge effluent (E1), et pour une autre partie en tête du premier réacteur (R1), et dans lequel le flux gazeux en provenance du compresseur de recycle (RCY) est envoyé en tête de l'avant dernier réacteur (R3) de la section réactionnelle.

Selon une première variante de la présente invention, l'effluent gazeux de l'étape de réduction est entièrement envoyé à l'entrée de l'échangeur charge effluent.

Selon une seconde variante de la présente invention, l'effluent gazeux de l'étape de réduction est entièrement envoyé en tête du premier réacteur.

Selon une troisième variante de l'invention, le flux gazeux en provenance du compresseur de recycle (RCY) est intégralement envoyé en tête du troisième réacteur.

Une variante qui combine l'une quelconque des variantes 1 et 2 concernant l'effluent gazeux de réduction, avec la variante 3 qui concerne le flux gazeux en provenance du compresseur de recycle (RCY), par exemple la variante 1 et la variante 3, ou la variante 2 et la variante 3 rentre dans le cadre de l'invention.

Plus précisément, dans une première configuration du procédé de reformage catalytique d'une essence selon la présente invention, l'effluent gazeux de réduction (4) est entièrement envoyé en tête du premier réacteur R1, et l'effluent (7) issu du compresseur de recycle (RCY) est entièrement recyclé à l'avant dernier réacteur (R3).

Dans une deuxième configuration du procédé de reformage catalytique d'une essence selon la présente invention, l'effluent gazeux de réduction (4) est entièrement envoyé à l'entrée de l'échangeur charge effluent (E1), et l'effluent (7) issu du compresseur de recycle (RCY) est entièrement recyclé à l'avant dernier réacteur (R3).

Plusieurs avantages techniques résultent de l'envoi de l'effluent de réduction pour partie à l'entrée de l'échangeur charge effluent, et pour partie en tête du premier réacteur:
Tout d'abord la diminution de la pression partielle d'hydrogène dans le premier réacteur conduit à une augmentation sensible de la production d'aromatiques et d'hydrogène par rapport au schéma conventionnel selon l'art antérieur. En effet, la diminution du ratio H₂/HC sur le réacteurs R1 permet d'améliorer les réactions de déshydrogénation des naphtènes dans lesdits réacteurs, et de réduire le craquage des paraffines longues.

Par ailleurs, la diminution de la pression partielle d'hydrogène sur le premier réacteur R1 a pour conséquence une diminution de la demande en utilités sur le compresseur de recycle (RCY) et, in fine, une réduction de la taille dudit compresseur compte tenu de la nouvelle pression de refoulement de ce dernier et/ou de la réduction du taux de recycle. En effet, la pression de refoulement du compresseur de recyclage (RCY) est grandement diminuée par rapport à l'art antérieur. La dite pression de refoulement est en effet sensiblement égale à la pression d'entrée du premier réacteur dans l'art antérieur et égale à la pression du dernier ou de l'avant dernier réacteur pour le procédé selon l'invention.

D'autre part, le recyclage de l'effluent du compresseur de recycle (RCY) arrivant en tête du dernier (R4) ou de l'avant dernier réacteur (R3), permet une augmentation sensible du ratio H₂/HC dans ces réacteurs dans lesquels est produit la majeure partie du coke.

Cet augmentation du ratio H₂/HC sur les derniers réacteurs R3 ou R4 permet soit de réduire le coke à régénérer soit, à iso coke, de diminuer le débit de gaz de recycle sur le dernier (R4) ou l'avant dernier réacteur (R3). Ainsi on obtient un gain sensible en utilité sur le compresseur de recycle (RCY).

Concernant la technologie de la section réactionnelle, deux cas sont possible:
- Les réacteurs R1, R2, R3, R4 sont disposés côte à côte, le catalyseur étant transporté du fond d'un réacteur (Rp) en tête du suivant (Rp+1), puis arrivé au dernier réacteur, il est amené vers la colonne de régénération par une ligne d'élévation,
- Les réacteurs R1, R2, R3 et R4 sont disposés en empilement vertical et le catalyseur s'écoule alors par gravité d'un réacteur (Rp) au suivant (Rp+1) situé au dessous de (Rp). Une ligne d'élévation en fond du dernier réacteur permet d'introduire le catalyseur en tête de la colonne de régénération.

La présente invention est parfaitement compatible avec ces deux technologies, l'une dite "côte à côte", l'autre dite "empilement".

### DESCRIPTION DÉTAILLÉE DE L'INVENTION

La description détaillée qui suit est faite en liaison avec la figure 1.

Une unité de reformage catalytique des essences (intervalle de distillation de 60°C à 250°C) comprend une section réactionnelle constituée d'au moins quatre réacteurs notés R1, R2, R3 et R4 (voir R5) travaillant en série, et une zone de régénération du catalyseur comprenant une étape (I) de combustion du coke déposé sur le catalyseur, une étape (II) d'oxychloration permettant de re-disperser les cristallites, et une étape (III) de réduction à l'hydrogène qui permet de réduire les oxydes du catalyseur avant sa réintroduction dans la zone réactionnelle. La zone réactionnelle est constituée de 4 réacteurs notés R1, R2, R3, R4 représentés sur la figure 1 en configuration "côte à côte".

La charge à traiter (1) traverse l'échangeur charge effluent (E1) puis le four de préchauffage (F1) avant d'être introduite dans le premier réacteur de la série R1 (flux 3).

Le catalyseur circule dans les différents réacteurs de la zone réactionnelle à l'état de lit mobile et est traversé de manière sensiblement radiale par la charge à traiter (3) et les différents effluents intermédiaires (8) à l'entrée de R2, (9) à l'entrée de R3 et (10) à l'entrée de R4.

L'effluent réactionnel en sortie du réacteur R4 est refroidi dans l'échangeur charge effluent (E1) puis dans un aeroréfrigérant et un échangeur avant d'être envoyé au ballon séparateur (BS) à partir duquel on extrait en tête le gaz dit "gaz de recycle" qui est envoyé au compresseur de recycle (RCY), et en fond un flux de molécules à plus de 4 atomes de carbone (noté C4+) qui constitue le reformat.

Le catalyseur est ensuite régénéré dans la zone de régénération qui comprend une étape de combustion du coke déposé sur le catalyseur (I), une étape d'oxychloration (II) et une étape de réduction à l'hydrogène (III).

Sur la figure 1 la zone de régénération n'est pas entièrement représentée. Seule est représentée la dernière étape de réduction du catalyseur (notée RED) au moyen d'un gaz riche en hydrogène (flux 4'). Ce flux (4') est en effet beaucoup plus riche en hydrogène (teneur supérieure à 90% molaire) que le gaz de recyclage (7) (teneur en hydrogène de 80% à 87% molaire).

Le catalyseur régénéré en sortie de l'étape de réduction (RED) est réintroduit en tête du premier réacteur R1.

L'étape de réduction du catalyseur alimenté par un flux riche en hydrogène (4') en provenance du ballon de recontactage haute pression (non représenté dans la figure 1) ou issu d'une purification d'hydrogène (comme les procédés PSA ou tout autres procédé connus de l'homme du métier), génère un gaz de réduction appelé effluent de réduction (4) dans la suite du texte. Selon l'art antérieur, cet effluent de réduction (4) est réintroduit en amont du compresseur de recycle (noté RCY), ou en amont du ballon séparateur (noté BS).

Dans la présente invention, cet effluent de réduction (4) est en partie recyclé en tête du premier réacteur R1 par le flux (6), et en partie recyclée à l'entrée de l'échangeur charge effluent (E1) par le flux (5).

Dans un cas particulier de la présente invention, l'effluent de réduction (4) est entièrement recyclé à l'entrée de l'échangeur charge effluent (E1).

Enfin, une partie de l'effluent (7) issu du compresseur de recycle, appelé gaz de recycle, est envoyé en partie en tête du dernier réacteur R4 (flux 7b), et en partie en tête de l'avant dernier réacteur R3 de la zone réactionnelle (flux 7a).

Dans une variante préférée de la présente invention, l'effluent (7) issu du compresseur de recycle (RCY) est intégralement envoyé en tête de l'avant dernier réacteur R3 (flux 7a).

Une variante de l'invention, consiste à utiliser le concept de circulation en parallèle ou hybride tel que décrit dans le brevet US7285205 de la demanderesse. Ce type de circulation est également valable pour les deux agencement de réacteurs proposés, c'est à dire la configuration "cote à cote" ou "empilé".

L'hydrogène en sortie de l'étape de réduction (RED), nommé effluent de réduction (4) a généralement les caractéristiques suivantes :
Pression : 5,2 bar effectifs (1 bar = 10⁵ pascal) à plus ou moins 0,5 bar,
Température : 450 - 520°C
Teneur en hydrogène : 90% à 99,9% volume,
Teneur en chlore : 20-50 ppm volume,
Teneur en eau : 50-100 ppm volume,
Pression d'entrée au premier réacteur : 4,9 bar effectif.

### EXEMPLES:

On compare deux exemples, l'un selon l'art antérieur et l'autre selon l'invention. L'exemple selon l'invention n'est en aucun cas limitatif des conditions opératoires de la présente invention. Il permet seulement de mettre en valeur les avantages résultants de la présente invention par comparaison avec l'exemple selon l'art antérieur.

Les conditions opératoires communes à l'exemple selon l'art antérieur et à l'exemple selon l'invention sont les suivantes:

### Charge:

Débit de charge : 75 tonnes/heure
Intervalle de distillation: 60° à 250°C
PNA (%vol) : 53 / 41 / 6
Octane moteur = 102

### Conditions opératoires:

Vitesse massique spatiale : 2h⁻¹
Taux de recycle: débit molaire d'H2 (7) en provenance du compresseur de recyclage (RCY) / débit molaire de charge HC fraiche (1):
   - égal à 1,5 et envoyé vers R1 selon l'art antérieur
   - égal à 1,5 et envoyé vers R3 selon l'invention

Température d'entrée réacteurs R1, R2, R3, R4: 515°C
a) Selon l'art antérieur le gaz de recyclage en provenance du ballon séparateur basse pression (BS) est envoyé en amont de l'échangeur charge /effluent qui précède le premier réacteur R1. Le rapport H2/HC envoyé au réacteur R1 est de 1,5 (mole/mole).
b) Selon la présente invention, dans une de ses configurations, le gaz de recyclage issu du ballon séparateur (BS) est envoyé à l'entrée de l'avant dernier réacteur R3 (flux 7a), et l'effluent de réduction est envoyé en tête du premier réacteur R1 (flux 6).

Le rapport H2/HC envoyé au réacteur R3 est de 1,5 (mole/mole).

Il en résulte un bilan pression au niveau du compresseur de recyclage (RCY) complètement différent.
- Selon l'art antérieur : le différentiel de pression compte tenu des pertes de charge du circuit et des différents éléments qui le constitue (fours, réacteurs, lignes, échangeurs, instrument de contrôle) est de 3,5 bars entre l'aspiration et le refoulement. La consommation du compresseur est de 3620 kW.
- Selon l'invention, ce différentiel de pression n'est plus que de 1,9 bars, ce qui limite fortement le dimensionnement et la consommation d'utilités dudit compresseur de recyclage (RCY). Le taux de compression du compresseur de recycle (RCY) passe donc de 2,1 selon l'art antérieur à 1,6 selon l'invention.

La consommation du compresseur est de 2250 kW, soit une réduction de pratiquement 40% par rapport à l'art antérieur.
1) Le recyclage de l'effluent de réduction (flux 6) en tête du premier réacteur R1 permet la réadsoprtion des composés chlorés sur le catalyseur présent dans les réacteurs R1 ou R2. Cet effet de réadsorption des composés chlorés permet également de réduire d'environ 30% la consommation de chlore dans la boucle de régénération du catalyseur.
2) Le recyclage de l'effluent de réduction (flux 6) en tête de R1 permet également d'améliorer sensiblement la sélectivité du catalyseur puisque le rendement en coupe C5+ passe de 90,1% selon l'art antérieur à 91,3% selon l'invention.
3) Enfin, le recyclage de l'effluent de réduction (flux 6) en tête du réacteur R1, permet de diminuer, (voire de supprimer complètement dans certains cas) l'injection d'eau dans la charge. Dans le cas présent, cette injection est diminuée de 4 ppm poids à 1,4 ppm poids.

Les performances de l'unité selon l'art antérieur et selon la présente invention sont résumées dans le tableau 1 ci dessous.

**TABLEAU 1**

| | Art antérieur | Selon invention | unités |
|---|---|---|---|
| H2/HC (envoyé au réacteur R1) | 1,5 | 0 | mol/mol |
| H2/HC (envoyé au réacteur R3) | 0 | 1,5 | mol/mol |
| C5+ | 90,1 | 91,3 | % pds |
| H2 | 3,6 | 3,9 | % pds |
| C1-C4 | 6,3 | 4,8 | % pds |
| Consommation du compresseur de recycle (RCY) | 3620 | 2250 | kW |
| Taux de compression du compresseur de recyclage | 2,1 | 1,6 | |
| Différentiel de pression du compresseur de recyclage | 3,5 | 1,9 | bars |
| H₂O alimentation | 4 | 1,4 | ppm poids |
| Pertes en chlore | base | -30% | relatif |

## Revendications

1. Procédé de reformage catalytique d'une charge d'essence d'intervalle de distillation compris entre 60°C et 250°C dans une unité de reformage catalytique en lit mobile comprenant une zone réactionnelle à quatre réacteurs en série (R1, R2, R3 et R4) de manière à produire un effluent réactionnel, et une zone de régénération dudit catalyseur comprenant une étape de réduction du catalyseur par de l'hydrogène,
dans lequel la charge traverse un échangeur charge effluent (E1) avant d'être introduite dans le premier réacteur (R1),
dans lequel l'effluent réactionnel en sortie du quatrième réacteur (R4) est refroidi dans l'échangeur charge effluent (E1),
dans lequel un effluent gazeux (4) de ladite étape de réduction du catalyseur est recyclé selon l'une des trois variantes suivantes :
- ledit effluent gazeux est recyclé en partie à l'entrée de l'échangeur charge effluent (E1) précédent le premier réacteur (R1) de la série, et en partie directement en tête du premier réacteur (R1),
- ledit effluent gazeux est entièrement envoyé en tête du premier réacteur (R1),
- ledit effluent gazeux est entièrement envoyé à l'entrée de l'échangeur charge effluent (E1),
et dans lequel un effluent de recycle (7) issu d'un compresseur de recycle (RCY) situé entre un ballon séparateur (BS) et la série des réacteurs (R1, R2, R3 et R4) est entièrement recyclé en tête de l'avant dernier réacteur (R3) ou est recyclé en partie en tête de l'avant dernier réacteur (R3) et en partie en tête du dernier réacteur (R4).

2. Procédé de reformage catalytique d'une essence selon la revendication 1, dans lequel l'effluent de réduction gazeux (4) est entièrement envoyé en tête du premier réacteur R1, et l'effluent (7) issu du compresseur de recycle (RCY) est entièrement recyclé à l'avant dernier réacteur (R3).

3. Procédé de reformage catalytique d'une essence selon la revendication 1, dans lequel l'effluent de réduction gazeux (4) est entièrement envoyé à l'entrée de l'échangeur charge effluent (E1), et l'effluent (7) issu du compresseur de recycle (RCY) est entièrement recyclé à l'avant dernier réacteur (R3).

## Patentansprüche

1. Verfahren zum katalytischen Reformieren einer Benzincharge mit einem Destillationsintervall im Bereich zwischen 60 °C und 250 °C in einer katalytischen Bewegtbett-Reformierungseinheit, umfassend eine Reaktionszone mit vier Reaktoren in Reihe (R1, R2, R3 und R4), um einen Reaktionsabstrom herzustellen, und eine Zone zur Regeneration des Katalysators, die einen Schritt zur Katalysatorreduktion mit Wasserstoff umfasst,
wobei die Charge einen Chargen-Abstrom-Tauscher (E1) durchläuft, bevor sie in den ersten Reaktor (R1) eingeführt wird,
wobei der Reaktionsabstrom am Auslass des vierten Reaktors (R4) in dem Chargen-Abstrom-Tauscher (E1) abgekühlt wird,
wobei ein gasförmiger Abstrom (4) des Schritts zur Katalysatorreduktion gemäß einer der folgenden drei Varianten zurückgeführt wird:
- der gasförmige Abstrom wird teilweise zum Einlass des Chargen-Abstrom-Tauschers (E1), der dem ersten Reaktor (R1) der Reihe vorausgeht, und teilweise direkt zum Kopf des ersten Reaktors (R1) zurückgeführt,
- der gasförmige Abstrom wird vollständig zum Kopf des ersten Reaktors (R1) geschickt,
- der gasförmige Abstrom wird vollständig zum Einlass des Chargen-Abstrom-Tauschers (E1) geschickt,
und wobei ein Rückführungsabstrom (7), der aus einem Rückführungskompressor (RCY) stammt, der zwischen einem Abscheiderbehälter (BS) und der Reihe der Reaktoren (R1, R2, R3 et R4) liegt, vollständig zum Kopf des vorletzten Reaktors (R3) oder teilweise zum Kopf des vorletzten Reaktors (R3) und teilweise zum Kopf des letzten Reaktors (R4) zurückgeführt wird.

2. Verfahren zum katalytischen Reformieren eines Benzins nach Anspruch 1, wobei der gasförmige Reduktionsabstrom (4) vollständig zum Kopf des ersten Reaktors R1 geschickt wird und der Abstrom (7), der aus dem Rückführungskompressor (RCY) stammt, vollständig zum vorletzten Reaktor (R3) zurückgeführt wird.

3. Verfahren zum katalytischen Reformieren eines Benzins nach Anspruch 1, wobei der gasförmige Reduktionsabstrom (4) vollständig zum Kopf des ersten Reaktors R1 geschickt wird und der Abstrom (7), der aus dem Rückführungskompressor (RCY) stammt, vollständig zum vorletzten Reaktor (R3) zurückgeführt wird.

## Claims

1. A process for catalytic reforming of a gasoline feed with a distillation range in the range 60°C to 250°C in a moving bed catalytic reforming unit comprising a reaction zone having four reactors in series (R1, R2, R3 and R4), so as to produce a reaction effluent, and a zone for regeneration of said catalyst comprising a step for reduction of catalyst by hydrogen,
in which the feed passes through an feed/effluent exchanger (E1) before being introduced in the first reactor (R1),
in which the reaction effluent at the outlet of the fourth reactor (R4) is cooled in the feed/effluent exchanger (E1),
in which a gaseous effluent (4) from said catalyst reduction step is recycled according to one of the three following possibilities :
- said gaseous effluent (4) is recycled in part to the inlet of the feed/effluent exchanger (E1) preceding the first reactor (R1) of the series, and in part directly to the head of reactor (R1),
- said gaseous effluent (4) is entirely sent to the head of the first reactor (R1),
- said gaseous effluent (4) is entirely send to the inlet of the feed/effluent exchanger (E1),
and in which the recycle effluent (7) coming from a recycle compressor (RCY) located between a separator drum (BS) and the series of reactors (R1, R2, R3 and R4) is recycled in its entirety to the head of the penultimate reactor (R3), or is recycled in part to the head of the penultimate reactor (R3) and in part to the head of the last reactor (R4).

2. A process for catalytic reforming of a gasoline according to claim 1, in which the gaseous reduction effluent (4) is sent in its entirety to the head of the first reactor (R1), and the effluent (7) coming from the recycle compressor (RCY) is recycled in its entirety to the penultimate reactor (R3).

3. A process for catalytic reforming of a gasoline according to claim 1, in which the gaseous reduction effluent (4) is sent in its entirety to the inlet of the feed/effluent exchanger (E1), and the effluent (7) coming from the recycle compressor (RCY) is recycled in its entirety to the penultimate reactor (R3).
